# EUROPÄISCHE PATENTANMELDUNG

(11) **EP 2 275 466 A1**
(43) Veröffentlichungstag der Anmeldung: **19.01.2011**
(21) Anmeldenummer: 09009248.7
(22) Anmeldetag: 16.07.2009
(51) Int. Cl.: C08G 18/10, C08G 18/18, C09J 175/12, A61L 24/04

(54) **Polyharnstoff-basierter Gewebekleber**

(71) Anmelder: Bayer MaterialScience AG, 51368 Leverkusen (DE)
(72) Erfinder: Heckroth, Heike, Dr., 51519 Odenthal (DE); Nefzer, Hartmut, Dr., 50259 Pulheim (DE); Eggert, Christoph, Dr., 50733 Köln (DE)

(57) **Zusammenfassung**

Die vorliegende Erfindung betrifft ein Polyhamstoff-System, die Verwendung eines solchen Polyharnstoff-Systems zum Verschließen, Verbinden, Verkleben oder Abdecken von Zellgewebe und ein Dosiersystem mit zwei Kammern für ein solches Polyharnstoff-System. Das Polyharnstoff-System umfasst als Komponenten: A) ein isocyanatfunktionelle Prepolymer, das durch Umsetzung von aliphatischen Isocyanaten mit Polyolen erhältlich ist und B) einen aminofunktionelle Asparaginsäureester. Zu diesem System wird erfindungsgemäß Wasser und / oder tertiäres Amin zugesetzt, um die Umsetzungsgeschwindigkeit des Prepolymers mit dem aminofunktionelle Asparaginsäureester zu erhöhen und gleichzeitig die Aushärtungszeit zu vermindern.

## Beschreibung

Die vorliegende Erfindung betrifft ein Polyharnstoff-System, die Verwendung eines solchen Polyharnstoff-Systems zum Verschließen, Verbinden, Verkleben oder Abdecken von Zellgewebe und ein Dosiersystem mit zwei Kammern für ein solches Polyharnstoff-System.

Diverse Materialien, die als Gewebekleber eingesetzt werden, sind im Handel erhältlich. Hierzu gehören die Cyanacrylate Dermabond® (Octyl-2-Cyanoacrylat) und Histoacryl Blue® (Butyl-Cyanoacrylat). Voraussetzung für eine effiziente Klebung der Cyanacrylate sind trockene Untergründe. Bei starken Blutungen versagen derartige Klebstoffe.

Als Alternative zu den Cyanacrylaten stehen biologische Klebstoffe wie zum Beispiel BioGlue^{®}, eine Mischung aus Glutaraldehyd und Bovinem Serumalbumin, diverse kollagen- und gelatinebasierte Systeme (FloSeal®) sowie die Fibrinkleber (Tissucol) zur Verfügung. Diese Systeme dienen in erster Linie der Blutstillung (Hämostase). Neben den hohen Kosten zeichnen sich Fibrinkleber durch eine relative schwache Klebestärke und einen schnellen Abbau aus, so dass diese nur bei kleineren Verletzungen auf nicht gespanntem Gewebe verwendet werden können. Kollagen- und Gelatinebasierte Systeme wie FloSeal® dienen ausschließlich der Hämostase. Zudem besteht, da Fibrin und Thrombin aus humanem-, Collagen und Gelatine aus tierischem Material gewonnen werden, bei biologischen Systemen immer die Gefahr einer Infektion. Biologische Materialien müssen außerdem gekühlt gelagert werden, so dass ein Einsatz in der Notfallversorgung wie z. B. in Katastrophengebieten, bei militärischen Einsetzen etc. nicht möglich ist. Hier steht zur Behandlung traumatischer Wunden QuikClot® oder QuikClot ACS+™ zur Verfügung, welches ein mineralisches Granulat ist, das im Notfall in die Wunde gebracht wird und dort durch Wasserentzug zur Koagulation führt. Im Falle von QuikClot® ist dies eine stark exotherme Reaktion, die zu Verbrennungen führt. QuikClot ACS+™ ist eine Gaze, in die das Salz eingebettet ist. Das System muss zur Blutstillung fest auf die Wunde gedrückt werden.

Aus der EP 2 011 808 A1 ist die Herstellung und Verwendung von Polyharnstoff-Systemen als Gewebekleber grundsätzlich bekannt. Die hier offenbarten Systeme umfassen wenigstens zwei Komponenten. Dabei handelt es sich A) um ein isocyanatfunktionelle Prepolymer, das durch Umsetzung von aliphatischen Isocyanaten mit Polyolen erhältlich ist und B) um einen aminofunktionellen Asparaginsäureester. Zusätzlich oder alternativ zu diesen Komponenten können auch als Komponente C) Umsetzungsprodukte isocyanatfunktioneller Prepolymere mit Asparaginsäureestern gemäß Komponente B) enthalten sein. Gegebenenfalls können auch organische Füllstoffe D), die eine nach DIN 53019 gemessenen Viskosität bei 23°C im Bereich von 10 bis 6000 mPas aufweisen, vorhanden sein.

Der aminofunktionelle Asparaginsäureester hat die folgende allgemeine Formel 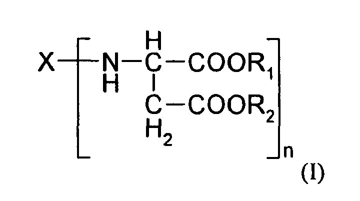 wobei X ein organischer Rest ist, der keinen Zerewitinoff-aktiven Wasserstoff aufweist, R₁, R₂ gleiche oder verschiedene organische Reste sind, die keinen Zerewitinoff-aktiven Wasserstoff aufweisen und n eine ganze Zahl von mindestens 2 ist.

Zur Definition von Zerewitinoff-aktivem Wasserstoff wird auf die entsprechende Definition in Römpp Chemie Lexikon, Georg Thieme Verlag Stuttgart verwiesen. In Rahmen dieser Anmeldung werden unter Gruppen mit Zerewitinoff-aktivem Wasserstoff insbesondere OH, NH oder SH verstanden.

Die offenbarten 2-Komponenten Polyharnstoff-Systeme können als Gewebekleber für den Verschluss von Wunden in menschlichen und tierischen Zellverbänden eingesetzt werden. Dabei kann ein sehr gutes Klebeergebnis erzielt werden. Allerdings wurde festgestellt, dass nicht bei allen offenbarten Systemen, insbesondere bei den nicht in den Ausführungsbeispielen erwähnten bioabbaubaren Polyester-basierten Systemen die Reaktionszeit der Umsetzung des Prepolymers mit dem aminofunktionellen Asparaginsäureester hoch genug ist, was zu unerwünscht langen Aushärtungszeiten von mehr als 6 min führt. Unter Aushärtungszeit wird dabei die Zeit verstanden, innerhalb der Gewebekleber zwei Gewebestücke so miteinander verbindet, dass diese nicht mehr durch leichten Zug voneinander getrennt werden können, bzw. die Zeit, nach der eine Blutung nach Auftragen des Gewebeklebers gestillt ist. Das Problem zu langer Aushärtungszeiten tritt insbesondere bei Systemen auf, deren Prepolymere auf Polyestern basieren. Eine geringe Aushärtungszeit von weniger als 2 min ist jedoch insbesondere dann notwendig, um beispielsweise einen hohen Blutverlust zu vermeiden. Die Systeme dürfen andererseits auch nicht zu schnell ausreagieren (< 20 s), um die Applizierbarkeit zu gewährleisten.

Aufgabe der Erfindung war es daher, das 2-Komponenten-System der EP 2 011 808 so zu verbessern, dass auch beim Einsatz von Ester basierten Prepolymeren eine hohe Umsetzungsgeschwindigkeit mit dem aminofunktionellen Asparaginsäureester und damit eine kurze Aushärtzeit realisiert werden kann. Dabei war als zusätzliche Bedingung zu beachten, dass das ausgehärtete System nach ISO 10993 bei der Anwendung im Menschen keine Zytotoxizität aufweisen darf.

Dieses Aufgabe wurde erfindungsgemäß dadurch gelöst, dass den aus der EP 2 011 808 A1 bekannten Polyharnstoff-Systemen Wasser und / oder tertiäres Amin zugesetzt wurde.

Es wurde festgestellt, dass bereits durch die alleinige Zugabe von entweder Wasser oder von tertiärem Amin die Umsetzungsgeschwindigkeit des Prepolymer mit dem aminofunktionellen Asparaginsäureester deutlich erhöht und damit die Aushärtzeit vermindert wird. Überraschender Weise steigt die Aushärtungszeit noch stärker an, wenn sowohl Wasser als auch tertiäres Amin zusammen zugesetzt werden. So wird die Umsetzungsgeschwindigkeit noch einmal um das ca.1, 3 fache erhöht, wenn eine Kombination von Wasser und tertiärem Amin an Stelle einer entsprechenden Menge nur einer der beiden Substanzen verwendet wird.

Gemäß einer bevorzugten Ausführungsform enthält das Polyharnstoff-System tertiäre Amine der allgemeinen Formel 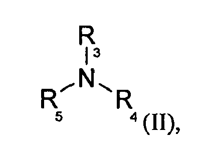 wobei R₃, R₄, R₅ unabhängig voneinander Alkyl- oder Heteroalkyreste mit Heteroatomen in der Alkylkette oder an deren Ende sein können, oder R₃ und R₄ gemeinsam mit dem sie tragenden Stickstoffatom einen aliphatischen, ungesättigten oder aromatischen Heterozyklus bilden können, der gegebenenfalls weitere Heteroatome enthalten kann.

Besonders bevorzugt sind tertiäre Amine ausgewählt aus der Gruppe Triethanolamin, Tetrakis (2-hydroxyethyl) ethylendiamin, N,N-Dimethyl-2-(4-methylpiperazin-1-yl)ethanamin, 2-{[2-(Dimethylamino)ethyl](methyl)amino}ethanol sowie 3,3',3"-(1,3,5-Triazinan-1,3,5-triyl)tris(N,N-dimethylpropan-1-amin).

Die Konzentration des Wassers im Polyharnstoffsystem kann insbesondere 0,2 bis 2,0 Gew.-% betragen. Bei der Zugabe von 0,2 Gew.-% Wasser erhöht sich die durchschnittliche Umsetzungsgeschwindigkeit um den Faktor 2. Wenn 2,0 Gew.-% Wasser zugesetzt werden, tritt eine Erhöhung der Umsetzungsgeschwindigkeit um den Faktor 8 auf.

Dem Polyharnstoff-System können insbesondere 0,1 bis 1,0 Gew.-% tertiäres Amin zugesetzt werden. Dabei bewirkt ein Zusatz von 0,1 Gew.% eine Verdoppelung der durchschnittlichen Umsetzungsgeschwindigkeit und die Zugabe von 1,0 Gew.-% führt zu einer Erhöhung um den Faktor 2,5.

Gemäß einer weiteren bevorzugten Ausführungsform ist X ein gegebenenfalls verzweigter oder linearer organischer Rest mit 1 bis 20, bevorzugt 2 bis 10 Kohlenstoffatomen und besonders bevorzugt ein C3, C4, C5 oder C6 Rest, der optional Heteroatome trägt.

Die Polyole A2) können bevorzugt Polyesterpolyole und/oder Polyester-Polyether-Polyole und/oder Polyetherpolyole, insbesondere mit einem Ethylenoxidanteil zwischen 60 und 90 Gew.-%, enthalten.

Vorzugsweise können die Polyole A2) ein zahlenmittleres Molekulargewicht von 2000 bis 8500 g/mol aufweisen.

Bei den organischen Füllstoffen der Komponente C) kann es sich bevorzugt um hydroxyfunktionelle Verbindungen, insbesondere um Polyetherpolyole handeln. Dabei können die Füllstoffe der Komponente C) eine mittlere OH-Funktionalität von 1,5 bis 3, bevorzugt von 1,8 bis 2,2 und insbesondere bevorzugt von 2 haben. Die Füllstoffe der Komponente C) können bei einer weiter bevorzugten Ausführungsform sich wiederholende Ethylenoxid Einheiten aufweisen.

Ein zweiter Aspekt der vorliegenden Erfindung betrifft die Verwendung der Polyharnstoff-Systeme zum Verschließen, Verbinden, Verkleben oder Abdecken von Zellgewebe, insbesondere zur Stillung des Austritts von Blut oder Gewebeflüssigkeiten oder dem Verschluss von Leckagen in Zellgewebe. So hat sich gezeigt, dass die erfindungsgemäßen Polyharnstoff-Systeme besonders gut zum Verbinden, Verkleben oder Abdecken von menschliches oder tierisches Zellgewebe geeignet sind. Mit ihrer Hilfe können schnell aushärtende, stark am Gewebe anhaftende, transparente, flexible und biokompatible Klebnähte hergestellt werden.

Gegenstand der Erfindung ist drittens ein Dosiersystem mit zwei Kammern für ein erfindungsgemäßes Polyharnstoff-System, dass in der einen Kammer die Komponente A) und in der anderen Kammer die Komponenten B) und E), sowie gegebenenfalls die Komponenten B, D + E sowie gegebenenfalls die Komponenten C) und D) mit evtl. E des Polyharnstoff-Systems enthält.

Die Herstellung der aminofunktionellen Polyasparaginsäureester B) erfolgt in bekannter Weise durch Umsetzung der entsprechenden primären mindestens difunktionellen Amine X(NH₂)ₙ mit Malein- oder Fumarsäureestern der allgemeinen Formel

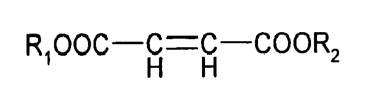

Bevorzugte Malein- oder Fumarsäureester sind Maleinsäuredimethylester, Maleinsäurediethylester, Maleinsäuredibutylester und die entsprechenden Fumarsäureester.

Bevorzugte primäre mindestens difunktionelle Amine X(NH₂)ₙ sind Ethylendiamin, 1,2-Diaminopropan, 1,4-Diaminobutan, 1,3-Diaminopentan, 1,5-Diaminopentan, 2-Methyl-1,5-diaminopentan, 1,6-Diaminohexan, 2,5-Diamino-2,5-dimethylhexan, 2,2,4- und/oder 2,4,4-Trimethyl-1,6-diaminohexan, 1,11-Diaminoundecan, 1,12-Diaminododecan, 1-Amino-3,3,5-trimethyl-5-aminomethyl-cyclohexan, 2,4-und/oder 2,6-Hexahydrotoluylendiamin, 2,4'-und/oder 4,4'-Diamino-dicyclohexylmethan, 3,3'-Dimethyl-4,4'-diamino-dicyclohexyl-methan, 2,4,4'-Triamino-5-methyl-dicyclohexylmethan und Polyetheramine mit aliphatisch gebundenen primären Aminogruppen mit einem zahlenmittleren Molekulargewicht Mₙ von 148 bis 6000 g/mol.

Besonders bevorzugte primäre mindestens difunktionelle Amine sind 1,3-Diaminopentan, 1,5-Diaminopentan, 2-Methyl-1,5-diaminopentan, 1,6-Diaminohexan, 1,13-Diamino-4,7,10-trioxatridecan. Ganz besonders bevorzugt ist 2-Methyl-1,5-diaminopentan.

In einer bevorzugten Ausführungsform der Erfindung sind R₁ = R₂ = Ethyl, wobei X auf 2-Methyl-1,5-diaminopentan als n-wertigem Amin basiert.

Die Herstellung der aminofunktionellen Asparaginsäureester A) aus den genannten Ausgangsmaterialien erfolgt nach DE-A 69 311 633 bevorzugt innerhalb des Temperaturbereichs von 0 bis 100 °C, wobei die Ausgangsmaterialien in solchen Mengenverhältnissen eingesetzt werden, dass auf jede primäre Aminogruppe mindestens eine, vorzugsweise genau eine olefinische Doppelbindung entfällt, wobei im Anschluss an die Umsetzung gegebenenfalls im Überschuss eingesetzte Ausgangsmaterialien destillativ abgetrennt werden können. Die Umsetzung kann in Substanz oder in Gegenwart geeigneter Lösungsmittel wie Methanol, Ethanol, Propanol oder Dioxan oder Gemischen derartiger Lösungsmittel erfolgen.

Die erfindungsgemäßen Systeme werden durch Mischung der Prepolymere A) mit den aminofunktionellen Asparaginsäureestern B) sowie gegebenenfalls den Komponenten C) und/oder D) erhalten. Das Verhältnis von freien oder blockierten Aminogruppen zu freien NCO-Gruppen beträgt bevorzugt 1:1,5, besonders bevorzugt 1:1. Wasser und/oder Amin werden dabei der Komponente B bzw. C beigemischt.

Die erfindungsgemäßen Systeme besitzen unmittelbar nach Vermischung der Einzelkomponenten miteinander eine nach DIN 53019 gemessene Scherviskosität bei 23°C von bevorzugt 500 bis 20000 mPas, besonders bevorzugt 500 bis 8000 mPas.

Die in A) eingesetzten isocyanatfunktionellen Prepolymere sind durch Umsetzung von Isocyanaten A1) mit Polyolen B2) gegebenenfalls unter Zusatz von Katalysatoren sowie Hilfs- und Zusatzstoffen erhältlich.

Als Isocyanate können beispielsweise monomere aliphatische oder cycloaliphatische Di- oder Triisocyanate wie 1,4-Butylendiisocyanat (BDI), 1,6-Hexamethylendiisocyanat (HDI), Isophorondiisocyanat (IPDI), 2,2,4- und/oder 2,4,4-Trimethylhexa-methylendiisocyanat, die isomeren Bis-(4,4'-isocyanatocyclohexyl)-methane oder deren Mischungen beliebigen Isomerengehalts, 1,4-Cyclohexylendiisocyanat, 4-Isocyanatomethyl-1,8-octandiisocyanat (Nonan-triisocyanat), sowie Alkyl-2,6-diisocyanatohexanoat (Lysindiisocyanat) mit C1-C8-Alkylgruppen eingesetzt werden.

Neben den vorstehend genannten monomeren Isocyanaten können auch deren höhermolekulare Folgeprodukte mit Uretdion-, Isocyanurat-, Urethan-, Allophanat-, Biuret-, Iminooxadiazindion- oder Oxadiazintrionstruktur sowie deren Mischungen eingesetzt werden.

Bevorzugt werden in A) Isocyanate der vorstehend genannten Art mit ausschließlich aliphatisch oder cycloaliphatisch gebundenen Isocyanatgruppen oder deren Mischungen eingesetzt.

Ganz besonders bevorzugt wird in A) Hexamethylendiisocyanat eingesetzt.

Die in A eingesetzten Polyole können sowohl Polyetherpolyole als auch Polyetherester sein. Die daraus synthetisierten Prepolymere sind bei Raumtemperatur flüssig und weisen eine nach DIN 53019 gemessene Scherviskosität bei 23°C von 200 bis 10.000 mPas, bevorzugt 400 bis 6000 mPas auf.

Solche Polyetheresterpolyole werden entsprechend dem Stand der Technik vorzugsweise durch Polykondensation aus Polycarbonsäuren, Anhydriden von Polycarbonsäuren, sowie Estern von Polycarbonsäuren mit leichtflüchtigen Alkoholen, bevorzugt C1 bis C6 Monoolen, wie Methanol, Ethanol, Propanol oder Butanol, mit molar überschüssigem, niedermolekularem und/oder höhermolekularem Polyol hergestellt; wobei als Polyol ethergruppenhaltige Polyole gegebenenfalls in Mischungen mit anderen ethergruppenfreien Polyolen eingesetzt werden.

Selbstverständlich können zur Polyetherestersynthese auch Gemische der höhermolekularen und der niedermolekularen Polyole verwendet werden.

Solche molar überschüssigen niedermolekularen Polyole sind Polyole mit Molmassen von 62 bis 299 Dalton, mit 2 bis 12 C-Atomen und Hydroxylfunktionalitäten von mindestens 2, die weiterhin verzweigt oder unverzweigt sein können und deren Hydroxylgruppen primär oder sekundär sind. Diese niedermolekularen Polyole können auch Ethergruppen aufweisen. Typische Vertreter sind Ethylenglykol, Propandiol-1,2, Propandiol-1,3, Butandiol-1,4, Butandiol-2,3, 2-Methyl-propandiol-1,3, Pentandiol-1,5, Hexandiol-1,6, 3-Methyl-pentandiol-1,5, 1,8-Octandiol, 1,10-Decandiol, 1,12-Dodecandiol, Cyclohexandiol, Diethylenglykol, Triethylenglykol und höhere Homologe, Dipropylenglykol, Tripropylenglykol und höhere Homologe, Glycerin, 1,1,1-Trimethylolpropan, sowie Oligo-tetrahydrofurane mit Hydroxylendgruppen. Selbstverständlich können innerhalb dieser Gruppe auch Gemische verwendet werden.

Molar überschüssige höhermolekulare Polyole sind Polyole mit Molmassen von 300 bis 3000 Dalton, die durch ringöffenende Polymerisation von Epoxiden, bevorzugt Ethylen- und/oder Propylenoxid, sowie durch säurekatalysierte, ringöffnende Polymerisation von Tetrahydrofuran, erhalten werden. Zur ringöffnenden Polymerisation von Epoxiden können entweder Alkalihdroxide oder Doppelmetallcyanidkatalysatoren verwendet werden.

Als Starter für ringöffnende Epoxidpolymerisationen können alle mindestens bifunktionellen Moleküle aus der Gruppe der Amine und der o.g. niedermolekularen Polyole verwendet werden. Typische Vertreter sind 1,1,1-Trimethylolpropan, Glycerin, o-TDA, Ethylendiamin, Propylenglykol-1,2, etc. sowie Wasser, einschließlich deren Gemische. Selbstverständlich können innerhalb der Gruppe der überschüssigen höhermolekularen Polyole auch Gemische verwendet werden.

Der Aufbau der höhermolekularen Polyole, soweit es sich um Hydroxylgruppen terminierte Polyalkylenoxide aus Ethylen- und/oder Propylenoxid handelt, kann statistisch oder blockweise erfolgen, wobei auch Mischblöcke enthalten sein können.

Polycarbonsäuren sind sowohl aliphatische als auch aromatische Carbonsäuren, die sowohl cyclisch, linear, verzweigt oder unverzweigt sein können und die zwischen 4 und 24 C-Atome aufweisen.

Beispiele sind Bernsteinsäure, Glutarsäure, Adipinsäure, Azelainsäure, Sebacinsäure, 1,10-Decandicarbonsäure, 1,12-Dodecandicarbonsäure, Phthalsäure, Terephthalsäure, Isophthalsäure, Trimellitsäure, Pyromellitsäure. Bevorzugt sind Bernsteinsäure, Glutarsäure, Adipinsäure, Sebacinsäure, Milchsäure, Phthalsäure, Terephthalsäure, Isophthalsäure, Trimellitsäure, Pyromellitsäure. Besonders bevorzugt sind Bernsteinsäure, Glutarsäure und Adipinsäure.

Weiterhin umfasst die Gruppe der Polycarbonsäuren auch Hydroxycarbonsäuren, bzw. deren innere Anhydride, wie z.B. Caprolacton, Milchsäure, Hydroxybuttersäure, Ricinolsäure, usw. Mit umfasst sind weiterhin auch Monocarbonsäuren, insbesondere solche, die über mehr als 10 C-Atome verfügen, wie Sojaölfettsäure, Palmölfettsäure und Erdnussölfettsäure, wobei deren Anteil am gesamten, das Polyetheresterpolyol aufbauenden Reaktionsmischung 10 Gew.-% nicht übersteigt und zusätzlich die dadurch einhergehende Minderfunktionalität durch Mitverwendung von mindestens trifunktionellen Polyolen, sei es auf Seiten der niedermolekularen oder der hochmolekularen Polyole ausgeglichen wird.

Die Herstellung der Polyetheresterpolyol erfolgt entsprechend dem Stand der Technik bei erhöhter Temperatur im Bereich von 120 bis 250°C, zunächst unter Normaldruck, später unter Anlegen von Vakuum von 1 bis 100 mbar, vorzugsweise, aber nicht notwendigerweise unter Verwendung eines Veresterungs- oder Umesterungskatalysators, wobei die Reaktion so weit vervollständigt wird, dass die Säurezahl auf Werte von 0,05 bis 10 mg KOH/g, bevorzugt 0,1 bis 3 mg KOH/g und besonders bevorzugt 0,15 bis 2,5 mg KOH/g absinkt.

Weiterhin kann im Rahmen der Normaldruckphase vor dem Anlegen von Vakuum ein Inertgas verwendet werden. Selbstverständlich können alternativ oder für einzelne Phasen der Veresterung auch flüssige oder gasförmige Schleppmittel zum Einsatz kommen. Beispielsweise kann das Reaktionswasser unter Verwendung von Stickstoff als Trägergas, ebenso ausgetragen werden, wie unter Einsatz eines Azeotropschleppmittels, wie z.B. Benzol, Toluol, Xylol, Dioxan, etc.

Selbstverständlich können auch Abmischungen von Polyetherpolyolen mit Polyesterpolyolen in beliebigen Verhältnissen eingesetzt werden.

Die eingesetzten Polyesterpolyole sind vorzugsweise hydrophil. Bevorzugt beträgt der auf Ethylenoxid zurückführbare Massenanteil der gesamten Komponente A bevorzugt 40 bis 95 Gew.-%, besonders bevorzugt 60 bis 90 Gew.-%.

Die eingesetzten Polyole haben bevorzugt eine mittlere OH-Funktionalität von 2 bis 4 und ein zahlenmittleres Molekulargewicht von 400 bis 20000 g/mol, besonders bevorzugt 2000 bis 10000 g/mol und ganz besonders bevorzugt 4000 bis 8500.

Polyetherpolyole sind bevorzugt Polyalkylenoxid-Polyether auf Basis von Ethylenoxid und gegebenenfalls Propylenoxid.

Diese Polyetherpolyole basieren bevorzugt auf di- oder höherfunktionellen Startermolekülen wie zwei- oder höherfunktionellen Alkoholen oder Aminen.

Beispiele solcher Starter sind Wasser (als Diol aufgefasst), Ethylenglykol, Propylenglykol, Butylenglykol, Glycerin, TMP, Sorbit, Pentaerythrit, Triethanolamin, Ammoniak oder Ethylendiamin.

Bevorzugte Polyalkylenoxid-Polyether entsprechen denen der vorstehend genannten Art und weisen Gehalt an Ethylenoxid-basierten Einheiten von 50 bis 100 %, bevorzugt 60 bis 90 % bezogen auf die insgesamt enthaltene Mengen an Alkylenoxideinheiten auf.

Ebenfalls können Hydroxylgruppen aufweisende Polycarbonate, bevorzugt Polycarbonatdiole, mit zahlenmittleren Molekulargewichten Mₙ von 400 bis 8000 g/mol, bevorzugt 600 bis 3000 g/mol eingesetzt werden. Diese sind durch Reaktion von Kohlensäurederivaten, wie Diphenylcarbonat, Dimethylcarbonat oder Phosgen, mit Polyolen, bevorzugt Diolen, erhältlich.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenol A und lactonmodifizierte Diole der vorstehend genannten Art in Frage.

Bevorzugt werden zum Prepolymeraufbau Polyetherpolyole der vorstehend genannten Art eingesetzt.

Zur Herstellung des Prepolymers werden das Isocyanat mit dem Polyol bei einem NCO/OH-Verhältnis von bevorzugt 4:1 bis 12:1, besonders bevorzugt 8:1 umgesetzt und anschließend der Anteil an nicht umgesetzten Isocyanates mittels geeigneter Methoden abgetrennt. Üblicherweise wird hierfür die Dünnschichtdestillation verwendet, wobei restmonomerenarme Produkte mit Restmonomergehalten von weniger als 1 Gew.-%, bevorzugt weniger als 0,1 Gew.%, ganz besonders bevorzugt weniger als 0,03 Gew.-% erhalten werden.

Gegebenenfalls können während der Herstellung Stabilisatoren wie Benzoylchlorid, Isophthaloylchlorid, Dibutylphosphat, 3-Chlorpropionsäure oder Methyltosylat zugesetzt werden.

Die Reaktionstemperatur beträgt dabei 20 bis 120°C, bevorzugt 60 bis 100°C.

Die hergestellten Prepolymere haben einen nach DIN EN ISO 11909 gemessenen mittleren NCO-Gehalt von 2 bis 10 Gew.-%, bevorzugt 2,5 bis 8 Gew.-%.

Die in D) eingesetzten organischen flüssigen Füllstoffe sind bevorzugt gemäß Zytotoxizitätsmessung nach ISO 10993 nicht zytotoxisch.

Beispielsweise können als organische Füllstoffe bei 23°C flüssige Polyethylenglykole wie PEG 200 bis PEG 600, deren Mono- bzw. Dialkylether wie PEG 500 Dimethylether, flüssige Polyether- und Polyesterpolyole, flüssige Polyester wie z.B. Ultramoll (Lanxess AG, Leverkusen, DE) sowie Glycerin und seine flüssigen Derivate wie z.B. Triacetin (Lanxess AG, Leverkusen, DE) eingesetzt werden.

Bevorzugt handelt es sich bei den organischen Füllstoffen der Komponente C) um hydroxy- oder aminofunktionelle, bevorzugt rein hydroxyfunktionelle Verbindungen. Besonders bevorzugt sind Polyole. Bevorzugte Polyole sind Polyether und/oder Polyesterpolyole, besonders bevorzugt Polyetherpolyole.

Die bevorzugten organischen Füllstoffe besitzen bevorzugt mittlere OH-Funktionalitäten von 1,5 bis 3, besonders bevorzugt 1,8 bis 2,2, ganz besonders bevorzugt 2,0.

Die bevorzugten organischen Füllstoffe der Komponente besitzen bevorzugt sich wiederholende von Ethylenoxid abgeleitete Einheiten.

Die Viskosität der organischen Füllstoffe beträgt bevorzugt 50 bis 4000 mPas, besonders bevorzugt 50 bis 2000 mPas bei 23°C gemessen nach DIN 53019.

In einer bevorzugten Ausführungsform der Erfindung werden als organische Füllstoffe Polyethylenglykole eingesetzt. Diese haben bevorzugt ein zahlenmittleres Molekulargewicht von 100 bis 1000 g/mol, besonders bevorzugt 200 bis 400 g/mol.

Das Gewichtsverhältnis der Füllstoffkomponente D) zur Aspartatkomponente B) beträgt 0 : 1 bis 20 : 1 , bevorzugt 0 : 1 bis 12 : 1.

Das Gewichtsverhältnis des Füllstoffes bezogen auf die Gesamtmenge der Mischung aus D und B liegt im Bereich von 0 bis 100 %, bevorzugt 0 bis 60 %.

Um das mittlere Equivalentgewicht der insgesamt zur Prepolymervernetzung eingesetzten Verbindungen bezogen auf die NCO-reaktiven Gruppen weiter zu reduzieren, ist es möglich zusätzlich zu den in A) eingesetzten Verbindungen auch die amino- oder hydroxyfunktionellen Umsetzungsprodukte isocyanatfunktioneller Prepolymere mit Asparaginsäureestern und/oder organischen Füllstoffen, sofern diese amino- oder hydroxyfunktionell sind, in einer separaten Vorreaktion herzustellen und dann als höhermolekulare Härterkomponente einzusetzen.

Bevorzugt werden bei der Vorverlängerung Verhältnisse von isocyanatreaktiven Gruppen zu Isocyanatgruppen von 50 zu 1 bis 1,5 zu 1 eingesetzt, besonders bevorzugt 15 zu 1 bis 4 zu 1.

Das dafür einzusetzende isocyanatfunktionelle Prepolymer kann dabei demjenigen der Komponente A) entsprechen oder aber auch abweichend aus den Komponenten, wie sie als mögliche Bestandteile der isocyanatfunktionellen Prepolymere im Rahmen dieser Anmeldung gelistet sind, aufgebaut sein.

Vorteil dieser Modifizierung durch Vorverlängerung ist, dass das Equivalentgewicht und Equivalentvolumen der Härterkomponente in deutlichen Grenzen modifizierbar ist. Dadurch können zur Applikation kommerziell verfügbare 2-Kammerdosiersysteme eingesetzt werden, um ein Klebesystem zu erhalten, das bei bestehenden Verhältnissen der Kammervolumina in das gewünschte Verhältnis von NCO-reaktiven Gruppen zu NCO-Gruppen eingestellt werden kann.

Selbstverständlich können in die Klebstofformulierung auch pharmakologisch aktive Wirkstoffe wie
a) Analgetika mit und ohne antiinflammatorische Wirkung
b) Antiphlogistika
c) Antimikrobiell wirksame Substanzen
d) Antimykotika
e) Antiparasitär wirkende Stoffe
eingearbeitet werden. Dies wird in der nicht vorveröffentlichten Patentanmeldung BMS 08 1 029 genauer beschrieben, was hiermit durch Verweis zum Gegenstand der vorliegenden Anmeldung gemacht wird.

Bezüglich weiterer für die Herstellung des Polyharnstoff-Systems geeigneten Komponenten und Herstellungsverfahren sowie der möglichen Verwendungen des Polyharnstoff-Systems wird die diesbezügliche Offenbarung der EP 2 011 808 A1 hiermit durch Verweis zum Gegenstand der vorliegenden Anmeldung gemacht.

### Beispiele

### PEG 600: Polyethylenglycol der Masse 600

Unter der Verarbeitungszeit wird die Zeit verstanden, innerhalb der die Polymermischung noch mittels eines Dosiersystems mit Statikmischer applizierbar ist.

### Beispiel 1 (Aspartat A)

Zu 2 Mol Diethylmaleat wurde unter Stickstoffatmosphäre langsam 1 Mol 2-Methyl-1,5-diaminopentan getropft, so dass die Reaktionstemperatur 60°C nicht überschritt. Anschließend wurde so lange auf 60°C erwärmt, bis kein Diethylmaleat mehr im Reaktionsgemisch nachweisbar war. Das Produkt wurde ohne weitere Reinigung eingesetzt.

### Beispiel 2 (Polyester A)

Aus einer gerührten Mischung bestehend aus 24.7 g Adipinsäure und 281.4 g PEG 600 wurden über 12h bei 210-220°C bei Atmosphärendruck Wasser abdestilliert. Anschließend wurden 30 mg Zinn(II)chlorid der Mischung zugegeben und unter Vakuum für weitere 24h Wasser abdestilliert, bis das Gemisch eine Säurezahl kleiner als 1 aufwies.

### Beispiel 3 (Polyester B)

Aus einer gerührten Mischung bestehend aus 24.7 g Adipinsäure und 152.3 g PEG 600 wurden über 12h bei 210-220°C bei Atmosphärendruck Wasser abdestilliert. Anschließend wurden 30 mg Zinn(II)chlorid der Mischung zugegeben und unter Vakuum für weitere 24h Wasser abdestilliert, bis das Gemisch eine Säurezahl kleiner als 1 aufwies.

Die Entwässerung der eingesetzten Polyester erfolgt durch einstündiges Rühren bei 80°C und 0.13 mbar.

### Beispiel 4 (Prepolymer A)

680 g HDI und 1.08 g Benzoylchlorid wurden in einem 1 1 Vierhalskolben vorgelegt. Innerhalb von 2h wurden bei 80°C 400 g des entwässerten Polyesters A hinzugefügt und eine Stunde nachgerührt. Anschließend wurde durch Dünnschichtdestillation bei 130°C und 0,13 mbar das überschüssige HDI abdestilliert. Man erhielt das Prepolymer mit einem NCO-Gehalt von 5.83%. Der Restmonomerengehalt betrug < 0,03 % HDI.

### Beispiel 5 (Prepolymer B)

240 g HDI und 0.53 g Benzoylchlorid wurden in einem 1 1 Vierhalskolben vorgelegt. Innerhalb von 2h wurden bei 80°C 287.3 g des entwässerten Polyesters B (1 h hinzugefügt und eine Stunde nachgerührt. Anschließend wurde durch Dünnschichtdestillation bei 130°C und 0,13 mbar das überschüssige HDI abdestilliert. Man erhielt das Prepolymer mit einem NCO-Gehalt von 3.83%. Der Restmonomerengehalt betrug < 0,03 % HDI.

### Beispiel 6

Zu 8 g des Prepolymers A wurden 2.6 g Aspartat A mit unterschiedlichen Mengen an Wasser und Amin gegeben und die Verarbeitungszeit gemessen.

| **Triethanolamin [%]** | **Wasser [%]** | **Verarbeitungszeit** |
|---|---|---|
| / | / | 11 min 20 s |
| / | 0.02 | 5 min 20 s |
| / | 0.2 | 1 min 40 s |
| 0.06 | / | 10 min 10 s |
| 0.6 | / | 9 min 50 s |
| 0.6 | 0.02 | 4 min 40 s |
| 0.6 | 0.2 | 1 min 15 s |

### Beispiel 7

Zu 8 g Prepolymer B wurden 1.71 g Aspartat A mit unterschiedlichen Mengen an Wasser und Amin gegeben und die Verarbeitungszeit gemessen.

| **Triethanolamin [%]** | **Wasser [%]** | **Verarbeitungszeit** |
|---|---|---|
| / | / | 20 min |
| / | 0.02 | 8 min 30 s |
| / | 0.2 | 2 min 20 s |
| 0.06 | / | 18 min |
| 0.6 | / | 13 min 50 s |
| 0.6 | 0.02 | 6 min 30 s |
| 0.6 | 0.2 | 2 min |

### Beispiel 8

Zu 8 g eines analog zu Prepolymer A hergestellten trifunktionellen Polyetherpolyol-HDI-Prepolymers mit einem NCO-Gehalt von 2.37 % wurden 1.08 g Aspartat A mit unterschiedlichen Mengen an Wasser und Amin gegeben und die Verarbeitungszeit gemessen.

| **Triethanolamin [%]** | **Wasser [%]** | **Verarbeitungszeit** |
|---|---|---|
| / | / | 9 min 50 |
| / | 0.02 | 5 min 40 s |
| / | 0.2 | 1 min 40 s |
| 0.06 | / | 6 min 50 s |
| 0.6 | / | 5 min 50 s |
| 0.6 | 0.02 | 4 min |
| 0.6 | 0.2 | 50 s |

In allen Fällen wurde keine Änderung der Klebestärke auf Muskelgewebe beobachtet.

## Patentansprüche

1. Polyharnstoff-System, umfassend
als Komponente A) isocyanatfunktionelle Prepolymere erhältlich durch Umsetzung von
aliphatischen Isocyanaten A1) mit
Polyolen A2), die ein zahlenmittleres Molekulargewicht von ≥ 400 g/mol und eine mittlere OH-Funktionalität von 2 bis 6 aufweisen
als Komponente B) aminofunktionelle Asparaginsäureester der allgemeinen Formel (I) 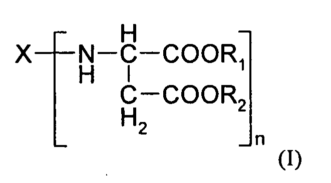 wobei
X ein organischer Rest ist, der keinen Zerewitinoff-aktiven Wasserstoff aufweist,
R₁, R₂ gleiche oder verschiedene organische Reste sind, die keinen Zerewitinoff-aktiven Wasserstoff aufweisen und
n eine ganze Zahl von mindestens 2 ist.
und/oder
gegebenenfalls als Komponente C) organische Füllstoffe, die insbesondere eine nach DIN 53019 gemessenen Viskosität bei 23°C im Bereich von 10 bis 6000 mPas aufweisen und
gegebenenfalls als Komponente D) Umsetzungsprodukte von isocyanatfunktionellen Prepolymeren gemäß der Definition der Komponente A) mit Asparaginsäureestern gemäß Komponente B) und/oder organischen Füllstoffen gemäß Komponente C)
**dadurch gekennzeichnet, dass** es
als Komponente E) Wasser und/oder tertiäres Am in enthält.

2. Polyharnstoff-System nach Anspruch 1, **dadurch gekennzeichnet, dass** es tertiäre Amine der allgemeinen Formel (II) 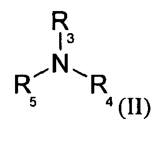 enthält, wobei
R₃, R₄, R₅ unabhängig voneinander Alkyl- oder Heteroalkyreste mit Heteroatomen in der Alkylkette oder an deren Ende sein können, oder R₃ und R₄ gemeinsam mit dem sie tragenden Stickstoffatom einen aliphatischen, ungesättigten oder aromatischen Heterozyklus bilden können, der gegebenenfalls weitere Heteroatome enthalten kann.

3. Polyharnstoff-System nach einem der Ansprüche 1 oder 2, **dadurch gekennzeichnet, dass** das tertiäre Amin ausgewählt aus der Gruppe Triethanolamin, Tetrakis (2-hydroxyethyl) ethylendiamin, N,N-Dimethyl-2-(4-methylpiperazin-1-yl)ethanamin, 2-{[2-(Dimethylamino)ethyl](methyl)amino}ethanol sowie 3,3',3"-(1,3,5-Triazinan-1,3,5-triyl)tris(N,N-dimethylpropan-1-amin) ist.

4. Polyharnstoff-System nach einem der Ansprüche 1 bis 3, **dadurch gekennzeichnet, dass** es 0,2 bis 2,0 Gew.-% Wasser und/oder 0,1 bis 1,0 Gew.-% tertiäres Amin enthält.

5. Polyharnstoff-System nach einem der Ansprüche 1 bis 4, **dadurch gekennzeichnet, dass** X ein gegebenenfalls verzweigter oder linearer organischer Rest mit 1 bis 20, bevorzugt 2 bis 10 Kohlenstoffatomen und besonders bevorzugt ein C3, C4, C5 oder C6 Rest ist, der optional Heteroatome trägt.

6. Polyharnstoff-System nach einem der Ansprüche 1 bis 5, **dadurch gekennzeichnet, dass** die Polyole A2) Polyesterpolyole und/oder Polyester-Polyether-Polyole und/oder Polyetherpolyole, insbesondere mit einem Ethylenoxidanteil zwischen 60 und 90 Gew.-%, enthalten.

7. Polyharnstoff-System nach Anspruch 6, **dadurch gekennzeichnet, dass** die zur Herstellung der Komponente A) eingesetzten Polyole ein zahlenmittleres Molekulargewicht von 4000 bis 8500 g/mol aufweisen.

8. Polyharnstoff-System nach einem der Ansprüche 1 bis 7, **dadurch gekennzeichnet, dass** es sich bei den organischen Füllstoffen der Komponente C) um hydroxyfunktionelle Verbindungen, insbesondere um Polyetherpolyole handelt.

9. Polyharnstoff-System nach Anspruch 8, **dadurch gekennzeichnet, dass** die Füllstoffe der Komponente C) eine mittlere OH-Funktionalität von 1,5 bis 3, bevorzugt von 1,8 bis 2,2 und insbesondere bevorzugt von 2 aufweisen.

10. Polyharnstoff-System nach einem der Ansprüche 8 oder 9, **dadurch gekennzeichnet, dass** die Füllstoffe der Komponente C) sich wiederholende Ethylenoxid Einheiten aufweisen.

11. Polyharnstoff-System nach einem der Ansprüche 1 bis 10 zum Verschließen, Verbinden, Verkleben oder Abdecken von Zellgewebe, insbesondere zur Stillung des Austritts von Blut oder Gewebeflüssigkeiten oder dem Verschluss von Leckagen in Zellgewebe.

12. Polyharnstoff-System nach Anspruch 11 zur Verwendung zum Verschließen, Verbinden, Verkleben oder Abdecken von menschliches oder tierisches Zellgewebe.

13. Verwendung eines Polyharnstoff-Systems nach einem der Ansprüche 1 bis 10 zur Herstellung eines Mittels zum Verschließen, Verbinden, Verkleben oder Abdecken von Zellgewebe, insbesondere zur Stillung des Austritts von Blut oder Gewebeflüssigkeiten oder dem Verschluss von Leckagen in Zellgewebe.

14. Verwendung nach Anspruch 13 zum Verschließen, Verbinden, Verkleben oder Abdecken von menschliches oder tierisches Zellgewebe.

15. Dosiersystem mit zwei Kammern für ein Polyharnstoff-System nach einem der Ansprüche 1 bis 12, **dadurch gekennzeichnet, dass** in der einen Kammer die Komponente A) und in der anderen Kammer die Komponenten B) und E), B), D) + E) sowie gegebenenfalls die Komponenten C) und D) und evtl. E) des Polyharnstoff-Systems enthalten sind.
